# EUROPÄISCHE PATENTSCHRIFT

(11) **EP 2 814 453 B1**
(45) Veröffentlichungstag und Bekanntmachung des Hinweises auf die Patenterteilung: **02.08.2017**
(21) Anmeldenummer: 13705121.5
(22) Anmeldetag: 07.02.2013
(51) Int. Cl.: A61K 8/39, A61Q 19/00, A61K 9/00, C07C 41/09, C07C 43/04

(54) **MISCHETHER ALS VASELINE-ERSATZ**
MIXED ETHER AS VASELINE SUBSTITUTE
ETHER MIXTE EN TANT QUE REMPLACEMENT DE LA VASELINE

(30) Priorität: 17.02.2012 US 201261599966 P; 17.02.2012 EP 12155966
(43) Veröffentlichungstag der Anmeldung: 24.12.2014
(73) Patentinhaber: BASF SE, 67056 Ludwigshafen (DE)
(72) Erfinder: DIERKER, Markus, 40593 Düsseldorf (DE); KEMPERS, Peter, 41189 Mönchengladbach (DE); KAWA, Rolf, 40789 Monheim (DE); BRÜNING, Stefan, 40593 Düsseldorf (DE); MILARDOVIC, Jadranka, 40591 Düsseldorf (DE); BECKEDAHL, Burkhard, 40589 Düsseldorf (DE); MAHNKE, Eike, Ulf, 42553 Velbert (DE); KRÜPPEL, Heinz-Josef, 41515 Grevenbroich (DE)
(74) Vertreter: BASF IP Association
(86) Internationale Anmeldenummer: PCT/EP2013/052425
(87) Internationale Veröffentlichungsnummer: WO 2013/120758

(56) Entgegenhaltungen:
- EP-A1- 0 970 998
- WO-A1-2004/006869
- DE-A1- 4 417 640

## Beschreibung

### Gebiet der Erfindung

Die vorliegende Erfindung betrifft Mischether, ihre Verwendung in kosmetischen und/oder pharmazeutischen Zubereitungen als Vaselineersatz, sowie kosmetische und/oder pharmazeutische Zubereitungen, enthaltend diese Mischether.

### Stand der Technik

Vaseline ist ein altbewährter Bestandteil vieler kosmetischer und/oder pharmazeutischer Grundlagen für die topische Anwendung. Sie findet eine sehr breite Anwendung in leave-on und rinse-off Zubereitungen, als Grundlage von Cremes und Salben und kann beispielsweise auch in Duschbädern eingesetzt werden.

Vaseline zählt zu den Kohlenwasserstoffgelen und stellt ein Zweiphasensystem mit 70 bis 90% einer flüssigen Phase aus n- und Isoparaffinen und Olefinkohlenwasserstoffen wie Ceten, Heptadecen und Octadecen, sowie 10 bis 30 % einer festen Phase. Die feste Phase besteht aus einem mikrokristallinen Anteil überwiegend von Isoparaffinen und geringen Anteilen Alicyclen und einem kristallinen Anteil aus n-Paraffinen. Die Gelstruktur der Vaseline entsteht durch die Ausbildung eines Gerüstes, das durch die längerkettigen festen Paraffine gebildet wird. Diese lagern sich - über van der Waals-London Kräfte gehalten - parallel aneinander und bilden sogenannte Fransenmicele, da die Enden der langketttigen Paraffine ungleichmäßig aus dem Micel herausragen und teilweise zur Bildung weiterer Micele beitragen. So entsteht ein dreidimensionales Gerüst durch zahlreiche miteinander verknüpfte Inseln parallel gelagerter langkettiger Paraffine, in das die flüssigen Kohlenwasserstoffe eingelagert werden. Die entsprechende Zusammensetzung von kristallinen, mikrokristallinen Bereichen und flüssigen Kohlenwasserstoffen bestimmt die Bildung dieser Gelstruktur und damit die besonderen rheologischen Eigenschaften (Plastizität, Duktilität) der Vaseline.
Vaseline zeichnet sich durch einen sehr breiten Schmelzbereich aus und verhält sich chemisch weitgehend neutral.

Überwiegend wird natürlich gewonnene Vaseline in kosmetischen und pharmazeutischen Zubereitungen verwendet, die ein bei der Erdölraffination im Rückstand anfallendes Gemisch von n-Paraffinen, Isoparaffinen und hydroaromatischen Kohlenwasserstoffen darstellt, das durch Behandlung mit konzentrierter Schwefelsäure und Bleicherden und/oder Aktivkohle aufgereinigt wird. Je nach Art der Aufreinigung entstehen unterschiedliche Qualitäten der Vaseline. Ebenso existiert aber auch eine synthetisch hergestellte Vaseline, die durch Auflösen von Paraffin und Ceresin in flüssigem Paraffin gewonnen wird.

Von Paraffinen ist jedoch bekannt, dass sie sich je nach Kettenlänge in Leber, Lymphknoten und Niere anreichern können. Immer wieder wird diskutiert, dass Mineralöle als schwer abbaubare Fette zur Anreicherung im Körper führen und durch Verschluss der Hautporen die Hautatmung verschlechtern oder die Entwicklung von Akne fördern. Auch Lippenpflegestifte mit Mineralölen sind daher bereits in Kritik geraten.

Trotz der bekanntermaßen guten topischen Verträglichkeit von Paraffinen, besteht ein anhaltendes Interesse nach Zubereitungen, welche Salbengrundlagen enthalten, die sich durch die Gewinnung aus nachwachsenden Rohstoffen auszeichnen. In den Eigenschaften sollen sie denen der Vaseline entsprechen. Bisherige Grundlagen zum Ersatz von Vaseline haben relativ enge Schmelzbereiche, es wurde daher nach möglichen Ersatzgrundlagen mit einem vergleichbaren breiten Schmelzbereich gesucht.

Bereits vor einigen Jahren diente die Abmischung von Bienenwachs und Pflanzenöl als Vaselineersatz. Bienenwachs ist jedoch als natürliches Produkt nicht kurzfristig in großen Mengen herstellbar.

Aus der DE 4417640 A1 ist eine Salbengrundlage auf Kohlenwasserstoffbasis mit Vaselinestruktur bekannt. Es handelt sich dabei um eine Lösung von Polyethylen hoher oder niederer Dichte, Vinylestern oder -ethern oder kristallinen Paraffinen in niedermolekularen hydrophoben Vinylpolymeren.

WO 2004/006869 A1 beschreibt ein Produkt, das einen Applikator umfasst, der mit einer wässrigen Phase und einer Lipidphase auf verschiedene Weisen verbunden sein kann. Die Lipidphase kann aus Ölen und Fetten bestehen, wobei Fettalkohole erwähnt werden.

EP 0970998 A1 beschreibt Wachsester mit vaselineähnlicher Konsistenz. Diese Wachsester sind spezielle Dicarbonsäureester, die aus einwertigen Alkoholen und Dicarbonsäuren hergestellt werden

Aus der Internationalen Anmeldung WO 2007/107966 sind Deodorantzubereitungen bekannt, die als Vaselineersatz hydriertes Rizinusöl enthalten, das durch Rizinussamenöl, flüssige Fettalkohole und Pflanzenöle in seiner Viskosität erniedrigt wird. Es wird vermutet, dass aufgrund der höheren Polarität von hydriertem Rizinusöl Duftstoffe länger in diesen kosmetischen Grundlagen verbleiben als in den Vaseline basierten Zubereitungen.

Die Synthese von Ethern aus Alkoholen ist ein seit langem etabliertes Verfahren in der chemischen Industrie. Kurzkettige Ether können als Lösemittel eingesetzt werden. Die Kondensation von primären Alkoholen kann z.B. durch Säuren oder Basen katalysiert werden, als Reaktionsprodukt entstehen Ether.

So sind unterschiedliche Ether auf dem Kosmetikmarkt verfügbar, z.B. Cetiol OE® von der BASF Personal Care and Nutrition GmbH.

Von der Firma Sasol sind unterschiedliche Cosmacol® Ether auf dem Markt ["Sasol Raw Materials for Cosmetics". Sasol Olefins & Surfactants GmbH Anckelmannsplatz 1, 20537 Hamburg, Germany], z.B. COSMACOL® OE (Dioctyl ether), COSMACOL® Ether 10 (Didecyl Ether), COSMACOL® Ether 12 (Dilauryl Ether), COSMACOL® Ether 14 (Dimyristyl Ether), COSMACOL® Ether 16 (Dihexadecyl ether) und COSMACOL® SE (Distearlyl ether) ["Sasol Raw Materials for Cosmetics". Sasol Olefins & Surfactants GmbH Anckelmannsplatz 1, 20537 Hamburg, Germany].

Von der Firma Nikko Chemicals ist ein Diisononyl Ether auf dem Markt (Nikkol DINE).

Diesen Ethern gemeinsam ist, dass sie nur reine Verbindungen mit definiertem Schmelzverhalten darstellen.

Einige kurzkettige Ether sind bei Raumtemperatur flüssig. Sie können beispielsweise als kosmetische Emollients eingesetzt werden. Längerkettige C18- Ether sind fest und haben einen hohen Schmelzpunkt im Bereich von > 45-50°C. Hochschmelzende Verbindungen haben in der Regel einen deutlich definierten Schmelzpunkt und sind somit nicht als Vaselineersatz geeignet.

### Beschreibung der Erfindung

Es wird ein Gemisch aus Ethern beschrieben, das in der Sensorik und den Anwendungseigenschaften mit Vaseline vergleichbar ist.

Die Erfindung betrifft Mischether, erhältlich durch Kondensationsreaktion von
a) 25 bis 90 Gew.% von unverzweigten Fettalkoholen mit einer Kettenlänge von 12 bis 18 Kohlenstoffatomen
b) 5 bis 50 Gew. % Isostearylalkohol
c) 0 bis 35 Gew. % eines Fettalkohols mit einer Kettenlänge von 20 und/oder 22 Kohlenstoffatomen und
d) 0 bis 25 Gew. % eines Fettalkohols mit 8 und/oder 10 Kohlenstoffatomen dadurch gekennzeichnet, dass sie einen nach dynamischer Differenzkalorimetrie (DSC) gemessenen Schmelzbereich zwischen -25 °C und +70 °C aufweisen, wobei die Breite des Schmelzbereichs mindestens 30 Temperaturgrade umfasst und das Maximum des Schmelzbereichs bei 35 ± 10 °C liegt.

Durch Optimierung der Fettalkoholzusammensetzung, Auswahl der Fettalkoholkettenlänge und Durchführung der Kondensationsreaktion können die Produkteigenschaften des Ethergemisches gezielt eingestellt werden, so dass das Gemisch den Anwendungseigenschaften und der Sensorik von Vaseline vergleichbar ist und einen vergleichbaren breiten Schmelzbereich mit einem ähnlichen Maximum aufweist.

Angaben des Schmelzbereiches von Vaseline in der Literatur liegen zwischen 35 und 60°C. Der Schmelzbereich variiert in Abhängigkeit vom Anteil der kristallinen Bereiche.
Durch Thermische Analyse mittels der Dynamischen Differenzkalorimetrie (DSC - englisch: Differential Scannning Calorimetry) kann jedoch bereits bei niedrigeren Temperaturen der Beginn des Schmelzens gemessen werden.

Die Tatsache, dass das Maximum des Schmelzbereiches bei Vaseline im Bereich der Hauttemperatur liegt und sich Vaseline durch einen relativ breiten Schmelzbereich mit einem langsamen Anstieg ab - 20 °C ± 5 °C bis 70 °C ± 5°C auszeichnet, hat einen wesentlichen Einfluss auf die charakteristischen sensorischen Eigenschaften von Vaseline.
Es wurde daher die Mischetherformulierung ausgewählt, die ein der Vaseline entsprechendes Schmelzverhalten aufweist.

### Charakterisierung des Schmelzbereiches:

Bei der dynamischen Differenzkalorimetrie (DSC) werden Wärmestrom-änderungen gemessen, die aufgrund von temperatur- oder zeitabhängigen Veränderungen der physikalischen und chemischen Struktur des Probenmaterials entstehen. Die DSC erfasst die Wärmeaufnahme des Probenmaterials für das Schmelzen der Probe bei gleichmäßig steigender Heizrate.

Die Ermittlung der Änderung der Wärmemenge kann messtechnisch verschiedenartig erfolgen. Bei der heutigen DSC (Differential Scanning Calorimetry) wird zwischen Wärmestrom-Differenz-Scanning-Kalorimetrie und der leistungskompensierten Differenz-Scanning-Kalorimetrie unterschieden.

Für die Messungen nach der Wärmestrom-Differenz-Scanning-Kalorimetrie (Tabellen 1a und b) wurde das Wärmestrom-DSC Q100 der Firma TA Instruments (Waters GmbH) verwendet. Es wurden jeweils fünf bis zehn Milligramm des Probenmaterials in Aluminium- Pfännchen eingewogen und hermetisch verkapselt (kaltverschweißt). Diese Pfännchen wurden mit einer Heizrate von 5 K/min einem Temperatur-Programm von - 80 °C bis + 100 °C unterworfen und das Schmelzverhalten bzw. Kristallisierverhalten analysiert. Die Ergebnisse wurden reproduzierbar gemessen.

Die Auswertung von Proben mit einer nicht konstanten Basislinie (aufgrund der Temperaturabhängigkeit der Wärmekapazität der Mischungen) und einem sehr breiten Schmelzbereich ist mit üblichen optischen Mitteln durch Ablesen der Werte aus den erhaltenen Diagrammen starken Schwankungen unterworfen, so dass die Temperaturwerte anhand von Enthalpiewerten festgelegt wurden.

Es wurde eine lineare Peak-Auswertung ab - 60 °C bis zum Ende des Aufschmelzens (lag zwischen + 40 °C und + 80 °C liegen) durchgeführt. Die so berechnete Enthalphie wurde prozentual betrachtet. Dadurch können Schmelzbereiche angegeben werden, indem die Temperatur bei 5 % der gesamten Schmelzenthalpie als Startpunkt und bei 99 % der gesamten Schmelzenthalpie als Ende des Bereiches gewählt wurde (Tabelle 1a). Diese Auswahl entsprach den ungefähr aus dem Diagramm optisch ermittelten Werten.

Als Temperaturmaximum wurde der Temperaturwert ermittelt, der sich bei maximalem Peak ergab. Dieser konnte mit ausreichender Genauigkeit aus den Diagrammen abgelesen werden (Tabelle 1b - rechte Spalte)

**Tab. 1a: - Enthalpie - Temperaturaufzeichnung der DSC-Messung**

| | | | | **1%** | | **5%** | | **10%** | | **50%** | | **90%** | | **95%** | | **99%** | |
|---|---|---|---|---|---|---|---|---|---|---|---|---|---|---|---|---|---|
| | **E in J/g gesamt** | **T (α) in °C** | **T (Ω) in °C** | **E in J/g** | **T in °C** | **E in J/g** | **T in °C** | **E in J/g** | **T in °C** | **E in J/g** | **T in °C** | **E in J/g** | **T in °C** | **E in J/g** | **T in °C** | **E in J/g** | **T in °C** |
| **A 75-25** | 149 | -60 | 47 | 1 | -35 | 7 | **-16** | 15 | -5 | 74 | 23 | 134 | 34 | 141 | 35 | 147 | **37** |
| **B 90-10** | 177 | -60 | 54 | 2 | -19 | 9 | **4** | 18 | 15 | 88 | 28 | 159 | 37 | 168 | 38 | 175 | **39** |
| **C 33-33-33** | 149 | -60 | 62 | 1 | -31 | 7 | **-10** | 15 | 1 | 75 | 33 | 134 | 49 | 142 | 51 | 148 | **53** |
| **D 25-25-50** | 120 | -60 | 60 | 1 | -40 | 6 | **-20** | 12 | -8 | 60 | 27 | 108 | 46 | 114 | 48 | 118 | **51** |
| **E 50-17-33** | 147 | -60 | 56 | 1 | -36 | 7 | **-17** | 15 | -5 | 73 | 27 | 132 | 42 | 140 | 44 | 145 | **47** |
| **F 70-05-25** | 157 | -60 | 51 | 2 | -34 | 8 | **-14** | 16 | -2 | 79 | 25 | 142 | 37 | 149 | 38 | 156 | **41** |
| **G 13-13-25-25-25** | 153 | -60 | 58 | 2 | -39 | 8 | **-21** | 15 | -12 | 77 | 23 | 138 | 42 | 145 | 46 | 152 | **49** |
| **Vaseline Hansen** | 73 | -60 | 76 | 1 | -44 | 3 | **-18** | 7 | -5 | 37 | 22 | 66 | 46 | 70 | 55 | 73 | **67** |
| **Vaseline Enzborn** | 64 | -60 | 76 | 1 | -48 | 3 | **-32** | 6 | -17 | 32 | 26 | 57 | 50 | 61 | 56 | 63 | **67** |
| **Vaseline Sigma Aldrich** | 63 | -60 | 76 | 1 | -49 | 3 | **-30** | 6 | -13 | 32 | 29 | 57 | 54 | 60 | 59 | 63 | **67** |
| **Vaseline VWR Prolabo** | 90 | -60 | 75 | 1 | -38 | 4 | **-10** | 9 | 0 | 45 | 24 | 81 | 47 | 85 | 56 | 89 | **67** |

**Tab. 1 b: Schmelzbereiche hergestellter Ethermischungen**

| | **Mischether** | **Mischungsverhältnis** | **Schmelzbereich (°C)** | **Schmelzbereichmaximum (°C)** |
|---|---|---|---|---|
| A | Lorol techn. / iso-C18-OH | 75:25 | - 16 - 37 | 37 |
| B | Lorol techn. / iso-C18-OH | 90:10 | 4 - 39 | 28 |
| C | Lorol techn. / Stenol 1822SR / iso-C18-OH | 33,3:33,3:33,3 | - 10 - 53 | 44 |
| D | Lorol techn. / Stenol 1822SR / iso-C18-OH | 25:25:50 | - 20 - 51 | 38 |
| E | Lorol techn. / Stenol 1822SR / iso-C18-OH | 50:16,7:33,3 | - 17 - 47 | 38 |
| F | Lorol techn. / Stenol 1822SR / iso-C18-OH | 70:5:25 | - 14 - 41 | 38 |
| G | Lorol C8 / Lorol C10 / Lorol techn. / Stenol 1822 SR / iso-C18-OH | 12,5:12,5:25:25:25 | -21 - 49 | 35 |
| Weisse Vaseline | unterschiedliche Qualitäten (4): Bezug: Sigma Aldrich / Hansen / VWR Prolabo/ Enzborn | | - 23 - 67 | 45 (Sigma) |
| | | | | 26 (Hansen) |
| | | | | 25 (Prolabo) |
| | | | | 35 (Enzborn) |

Die erfindungsgemäßen Mischether weisen einen durch dynamische Differenzkalorimetrie (DSC) gemessenen Schmelzbereich zwischen -25°C und +70°C auf, wobei die Breite des Schmelzbereichs mindestens 30 Temperaturgrade umfasst und das Maximum des Schmelzbereichs bei 35 ± 10 °C liegt.

Durch Schwankungen der Zusammensetzung von Vaseline insbesondere den unterschiedlichen Anteilen an kristallinen Bereichen variieren die mit der genauen Methode der DSC ermittelten Werte, so dass auch der für die erfindungsgemäßen Mischether ermittelte Schmelzbereich im Temperaturbereich zwischen -25 °C und +70 °C, bevorzugt zwischen -22°C und +55°C und besonders bevorzugt zwischen -20 °C und +55 °C liegt. Dabei muss sich der Schmelzbereich nicht über die gesamte Breite hinziehen, er sollte aber mindestens einen Bereich von 30 Temperaturgraden (°C) innerhalb des Temperaturbereiches zwischen -25 °C und +70 °C, bevorzugt innerhalb des Temperaturbereiches zwischen -22°C und +55°C und besonders bevorzugt innerhalb des Temperaturbereiches zwischen -20 °C und +55 °C abdecken, vorzugsweise soll er mindestens 50 Temperaturgrade (°C) und besonders bevorzugt mindestens 60 Temperaturgrade (°C) in seiner Breite ausmachen. Das Maximum des Schmelzbereiches liegt dabei bei 35 ± 10 °C, vorzugsweise bei 35 ± 5 °C und fällt somit ungefähr in den Bereich der Hauttemperatur.
Bevorzugt sind daher Mischether mit einem Schmelzbereich zwischen -22 °C und +55 °C, einer Breite des Schmelzbereichs von mindestens 50 Temperaturgraden und einem Maximum bei 35 ± 10 °C.

Besonders bevorzugt sind Mischether mit einem Schmelzbereich zwischen -20 °C und +55 °C, einer Breite des Schmelzbereichs von mindestens 60 Temperaturgraden und einem Maximum bei 35 ± 10 °C, sowie spezielle Mischether mit einem Schmelzbereich zwischen -20°C und +55 °C, einer Breite des Schmelzbereichs von mindestens 60 Temperaturgraden und einem Maximum bei 35 ± 5°C.

Überraschender Weise wurde gefunden, dass Mischether, dadurch gekennzeichnet, dass sie einen nach dynamischer Differenzkalorimetrie (DSC) gemessenen Schmelzbereich zwischen -25°C und +70°C aufweisen, wobei die Breite des Schmelzbereichs mindestens 30 Temperaturgrade umfasst und das Maximum des Schmelzbereichs bei 35 ± 10 °C liegt, erhältlich sind durch Kondensationsreaktion von
a) 25 bis 90 Gew.% von unverzweigten Fettalkoholen mit einer Kettenlänge von 12 bis 18 Kohlenstoffatomen
b) 5 bis 50 Gew. % Isostearylalkohol
c) 0 bis 35 Gew. % eines Fettalkohols mit einer Kettenlänge von 20 und/oder 22 Kohlenstoffatomen und
d) 0 bis 25 Gew. % eines Fettalkohols mit 8 und/oder 10 Kohlenstoffatomen.

Bevorzugt sind Mischether, die erhältlich sind durch Kondensationsreaktion von
a) 25 bis 70 Gew.% von unverzweigten Fettalkoholen mit einer Kettenlänge von 12 bis 18 Kohlenstoffatomen und
b) 5 bis 50 Gew. % Isostearylalkohol
c) 1 bis 35 Gew. % eines Fettalkohols mit einer Kettenlänge von 20 und/oder 22 Kohlenstoffatomen und
d) 0 bis 25 Gew. % eines Fettalkohols mit 8 und/oder 10 Kohlenstoffatomen.

Weiterhin bevorzugt sind Mischether, erhältlich durch Kondensationsreaktion von
a) 25 bis 50 Gew.% von unverzweigten Fettalkoholen mit einer Kettenlänge von 12 bis 18 Kohlenstoffatomen
b) 25 bis 50 Gew. % Isostearylalkohol
c) 15 bis 35 Gew. % eines Fettalkohols mit einer Kettenlänge von 20 und/oder 22 Kohlenstoffatomen und
d) gegebenenfalls 10 bis 25 Gew. % eines Fettalkohols mit 8 und/oder 10 Kohlenstoffatomen.

Besonders bevorzugt sind Mischether, erhältlich durch Kondensationsreaktion von
a) 25 bis 35 Gew.% von unverzweigten Fettalkoholen mit einer Kettenlänge von 12 bis 18 Kohlenstoffatomen und
b) 33 bis 50 Gew. % Isostearylalkohol
c) 25 bis 35 Gew. % eines Fettalkohols mit einer Kettenlänge von 20 und/oder 22 Kohlenstoffatomen.
Als Komponente a) werden unverzweigte, gesättigte Fettalkohole mit 12 bis 18 Kohlenstoffatomen eingesetzt. Um den Eigenschaften der Vaseline möglichst weitgehend zu entsprechen hat sich besonders eine Kettenverteilung unverzweigter Fettalkohole von: C12-alkohol von 48 - 58 %
C14-alkohol von 18 - 24 %
C16-alkohol von 8 - 12 %
C18-alkohol von 11 - 15 %
bewährt. Diese Zusammensetzung ist unter dem Handelsnamen Lorol® - "Lorol technisch" erhältlich. Sie weist einen Erstarrungsbereich von 18 bis 23 °C auf.

Komponente b) Isostearylalkohol (Prisorine 3515) - auch Isooctadecanol oder Isooctadecylalcohol - wird in der Kosmetik als Emollient eingesetzt mit einem guten Spreitungsvermögen. Aufgrund seiner guten Verträglichkeit wird dieser Alkohol auch bei der Synthese von Spezial-Surfactants verwendet. Handelsnamen sind: *Aldo 66; Emery 3389; Emery H 3600; FOC 1800; Fine Oxocol 180; Jarcol I 18EX; OHV 20; Prisorine 3515; Prisorine ISOH 3515;Risonol 18SP; Speziol C 18 ISOC; Speziol C 18 Iso; Witcohol 66*

Die Komponente c) besteht überwiegend aus Fettalkoholen mit einer Kettenlänge von 22 Kohlenstoffatomen, wobei diese gesättigt, ungesättigt, verzweigt oder geradlinig sein können. Vorzugsweise wird Behenylalkohol (synonym: n-Docosanol) eingesetzt, der mindestens 42 %, bevorzugt mindestens 65 %, besonders bevorzugt mindestens 70 % Fettalkohole der Kettenlänge C22 enthält. Fettalkoholzusammensetzungen dieser Art sind unter dem Namen Stenol® 1822 im Handel.

Die gegebenenfalls einzusetzende Komponente d) des C8-Fettalkohols, sowie des C10-Fettalkohols kann gesättigt, ungesättigt, verzweigt oder geradlinig sein. Vorzugsweise wird als C8-Fettalkohol jedoch n-Octanol (Octylalkohol), besonders bevorzugt in einer Qualität mit der Kettenverteilung von mindestens 95 % C8 eingesetzt. Als C10-Fettalkohol wird vorzugsweise n-Decanol (Decylalkohol), besonders bevorzugt mit einer Kettenverteilung von mindestens 90 % C10 Kettenanteil eingesetzt.

Die erfindungsgemäßen Mischether haben nicht nur einen vergleichbaren Schmelzbereich wie Vaseline, zeigen vergleichbare sensorische Eigenschaften wie Vaseline, sondern sie haben gegenüber Vaseline verbesserte Anwendungseigenschaften in tensidischen Systemen, da die Schaummenge in Rezepturen mit Mischethern größer ist als in vaselinehaltigen Zubereitungen.

Zwei ausgewählte Formulierungen eignen sich sehr gut für den Einsatz als Ersatz für Vaseline in kosmetischen und pharmazeutischen Zubereitungen, da sie einen sehr ähnlichen Schmelzbereich - zwischen -10 und 50 °C - aufweisen:
Die erste Formulierung ist erhältlich durch Umsetzung der Komponenten a), b) und c) zu gleichen Teilen. Besonders bevorzugt werden Zubereitungen, erhältlich durch die Umsetzung von Behenylalkohol, C12 bis C18 Fettalkoholgemisch (Lorol® technisch) und Isostearylalkohol im Verhältnis 33,3:33,3:33,3 .
Eine weitere Formulierung ist erhältlich durch die Umsetzung der Komponenten a), b) und c) im Verhältnis 25 : 25 : 50, wobei speziell die Zusammensetzung aus C12 bis C18 Fettalkoholen (Lorol® technisch), Behenylalkohol und Isostearylalkohol im Verhältnis 25:25:50 den gesuchten Schmelzpunktsbereich aufweist.

Eine dritte Zubereitung, die sich durch ihre hohe Okklusivität auszeichnet ist erhältlich durch Umsetzung der Komponenten a), b), c), d) im Verhältnis 25 : 25 : 25 : 25 wobei der C8- und der C10-Fettalkohol zu gleichen Teilen eingesetzt werden, speziell die Zusammensetzung aus Lorol® technisch, Behenylalkohol, Isostearylalkohol, C8-Fettalkohol und C10-Fettalkohol im Verhältnis 25 : 25 : 25 : 12,5 : 12,5 zeichnet sich durch sensorische Eigenschaften aus, die denen der Vaseline entsprechen.

### Synthesebedingungen für die Kondensationsreaktion:

### Bevorzugte Reaktionsbedingungen:

Die Veretherung geht von Fettalkoholen aus, die unter Säurekatalyse reagieren. Geeignete Säuren sind hier besonders starke Säuren, die Lewis und Brönsted sauer reagieren, aber auch Kombinationen mehrerer Säuren. Beispiele sind Sulfonsäure, Methansulfonsäuren und bevorzugt Trifluormethansulfonsäuren, auch in Kombination mit Phosphor-Säuren insbesondere Unterphosphorige Säure, besonders bevorzugt ist die Kombination von Trifluormethansulfonsäure und Unterphosphoriger Säure.
Die Reaktionstemperatur liegt über 150 °C, bevorzugt bei 200 bis 250°C, besonders bei 220°C ± 5°C.

Für die Umsetzung sind Alkohole mit Kettenlängen von C6 bis C36 möglich, die linear, verzweigt, gesättigt oder ungesättigt sind, wobei überwiegend mit Fettalkoholen mit einer Länge von 8 bis 22 Kohlenstoffatomen gearbeitet wird und diese vorzugsweise gesättigt und linear sind. Wesentlich ist die Auswahl der Mischungen, um die Eigenschaften des resultierenden Fettalkoholgemisches gezielt so einstellen zu können, dass sie denen der Vaseline entsprechen. Ziel ist ein bei Raumtemperatur halbfestes Material mit einem breiten Schmelzbereich.

Es können zur Vernetzung auch mehrwertige Alkohole mit 2 bis 36 Kohlenstoffatomen eingesetzt werden, vorzugsweise Guerbetalkohole und Diole, besonders bevorzugt Diole mit 6, 16 oder 36 Kohlenstoffatomen, da sie eine weitere Vernetzung der Alkohole bewirken. Zur Verbesserung der Farbe kann gebleicht werden, entstehende Nebenprodukte können per Destillation entfernt werden. Das Produkt selbst wird dabei nicht destilliert, da der Siedepunkt zu hoch ist. Es ist möglich und in der Regel erforderlich den sauren Katalysator vor der Destillation zu neutralisieren, dazu sind Alkali- und Erdalkali-Laugen geeignet.

Aufgrund der physikalischen, chemischen und insbesondere rheologischen Eigenschaften sind die Mischether zum Ersatz von Vaseline in kosmetischen oder pharmazeutischen Zubereitungen zu verwenden.

### Kosmetische Zubereitungen

Die erfindungsgemäßen Zubereitungen eignen sich als Basis in allen pharmazeutischen Zubereitungen zur topischen Anwendung und allen kosmetischen Mittel zur Körperpflege und -reinigung wie z.B. Körperöl, Babyöl, Körpermilch, Cremes, Lotionen, sprühbare Emulsionen, Sonnenschutzmittel und Antitranspirantien,. Sie lassen sich besonders in tensidhaltigen Zubereitungen wie z.B. Flüssig- und Stückseifen, Schaum- und Duschbädern, Haarshampoos und Pflegespülungen einsetzen. Möglich ist auch der Einsatz als Pflegekomponente auf Tissues, Papiere, Wipes, Vlies-Produkte, Schwämme, Puffs, Pflaster und Bandagen applizieren, die im Bereich der Hygiene und Pflege verbreitet sind (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte, Selbstbräunungs Wipes). Sie lassen sich u.a. auch in Zubereitungen zur Haarpflege, Haarreinigung oder Haarfärbung einsetzen. Sie lassen sich weiterhin in Zubereitungen der dekorativen Kosmetik, wie beispielsweise Lippenstiften, lipgloss, Make-up, Foundations, Puder, Lidschatten, Maskara und ähnlichem einsetzen.

Die Einsatzkonzentrationen in den jeweiligen Formulierungen und Zubereitungen entsprechen denen von Vaseline. Die pharmazeutischen und kosmetischen Zubereitungen enthaltend die erfindungsgemäßen Mischether, sind daher ebenfalls Gegenstand der Erfindung.

Da die erfindungsgemäßen Mischether insbesondere in tensidischen Zubereitungen Vorteile gegenüber dem Einsatz von Vaseline haben, dadurch dass die Schaummenge höher ist als in vergleichbaren vaselinehaltigen Systemen, sind auch kosmetische und/oder pharmazeutische Zubereitungen enthaltend die erfindungsgemäßen Mischether und grenzflächenaktive Substanzen Gegenstand der Erfindung.
Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Füllstoffe, Hydrotrope, Solubilisatoren, Konservierungsmittel, Parfümöle, Farbstoffe etc., die nachstehend exemplarisch aufgelistet sind.

Als grenzflächen-aktive Substanz eignet sich prinzipiell jede Substanz, welche die Oberflächenspannung zwischen der wässrigen und der nicht-wässrigen Phase erniedrigt. Grenzflächen-aktive Substanzen umfassen Emulgatoren und Tenside.

In einer Ausführungsform der Erfindung enthält die erfindungsgemäße Zubereitung mehr als eine grenzflächen-aktive Substanz. Der Fachmann setzt in Abhängigkeit der übrigen Komponenten übliche Systeme (wie z.B. Emulgator und Co-Emulgator) ein.

Ein geeigneter Emulgator ist prinzipiell jede grenzflächen-aktive Substanz, insbesondere jedoch Substanzen mit einem HLB-Wert von 1 bis 20 nach der Griffin Skala. Jedem Emulgator wird ein so genannter HLB-Wert (eine dimensionslose Zahl zwischen 1 und 20, Griffin Skala) zugeschrieben, der angibt, ob eine bevorzugte Wasser- oder Öllöslichkeit vorliegt. Zahlen unter 9 kennzeichnen bevorzugt öllösliche, hydrophobe Emulgatoren, Zahlen über 11 wasserlösliche, hydrophile Emulgatoren. Der HLB-Wert sagt etwas über das Gleichgewicht der Größe und Stärke der hydrophilen und der lipophilen Gruppen eines Emulgators aus.

Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Ölphase entsteht unter sonst gleichen Herstellungsbedingungen eine W/O-Emulsion.

### Nicht-ionische Emulgatoren,

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglyceryl-4-Laurate, Polyglycerindiisostearat oder Polyglycerindimerat. Ebenfalls geeignet sind Gemische von Verbindungen aus mehreren dieser Substanzklassen, wie z.B.Polyglyceryl-4 Diisostearate/Polyhydroxystearate/Sebacate
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester, sowie Sucrose Polystearate (kommerziell erhältlich als Emulgade® SUCRO, Cognis GmbH).
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls^{®} PGPH" (W/O-Emulgator) oder "Eumulgin^{®} VL 75" (Abmischung mit Coco Glucosides im Gewichtsverhältnis 1:1, O/W-Emulgator) oder Dehymuls^{®} SBL (W/O-Emulgator) von der Cognis Deutschland GmbH vertrieben werden. In diesem Zusammenhang sei insbesondere auf das Europäische Patent EP 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitan-dihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitan-trimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8- 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12, Ceteareth-20 und PEG-20 Stearat. Als Solubilisatoren bevorzugt geeignet sind Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether), sowie Eumulgin^{®} SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und - oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 6 bis 24, vorzugsweise 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} oder Plantaren® zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade^{®} PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin^{®} VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Emulgatoren können beispielsweise Silikonemulgatoren enthalten sein. Diese können beispielsweise aus der Gruppe der Alkylmethicon-copolyole und/oder Alkyl-Dimethiconcopolyole gewählt werden, insbesondere aus der Gruppe der Verbindungen, welche gekennzeichnet sind durch die folgende chemische Struktur: 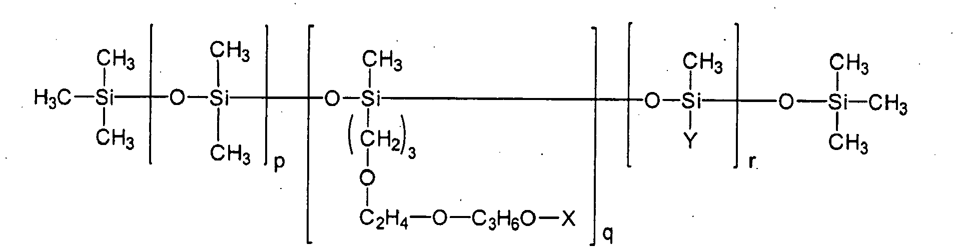 bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendenden Silikonemulgatoren sind Dimethiconcopolyole, welche von Evonik Goldschmidt unter den Warenbezeichnungen AXIL® B 8842, ABIL® B 8843, ABIL® B 8847, ABIL® B 8851, ABIL® B 8852, ABIL® B 8863, ABIL® B 8873 und ABIL®B 88183 verkauft werden.

Ein weiteres Beispiel für besonders vorteilhaft im Sinne der vorliegenden Erfindung zu verwendende grenzflächenaktiven Substanzen ist das Cetyl PEG/PPG-10/1 Dimethicone (Cetyl Dimethiconcopolyol), welches von EvonikGoldschmidt unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopo- lyol, welches von Evonik Goldschmidt unter der Warenbezeichnung ABIL®EM 97 und ABIL®WE 09 verkauft wird.

Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning® 5200 Formulation Aid von der Gesellschaft Dow Corning Ltd. erhältlich ist. Weiterhin vorteilhaft ist ein Silikonemulgator mit der INCI Bezeichnung Cyclopentasiloxane and PEG/PG-18-18 Dimethicone", der beispsielsweise unter dem Handelsnamen Dow Corning® 5225 C Formulation Aid erhältlich ist.

Ein weiterer vorteilhafter Silikonemulgator ist Octyl Dimethicon Ethoxy Glucosid der Firma Wacker. Für eine erfindungsgemäße Wasser-in-Silikonöl-Emulsion können alle bekannten für diesen Emulsionstyp verwendeten Emulgatoren eingesetzt werden. Erfindungsgemäß besonders bevorzugte Wasser-in-Silikon-Emulgatoren sind dabei Cetyl PEG/PPG- 10/1 Dimethicone und Lauryl PEG/PPG-18/18 Methicone [z.B. ABIL® EM 90 Evonik Goldschmidt), DC5200 Formulation Aid (Dow Corning)] sowie beliebige Mischungen aus beiden Emulgatoren.

Ein geeigneter anionischer O/W Emulgator ist z.B das unter der INCI Bezeichung erhältliche Produkt Disodium Cetearyl Sulfosuccinate (Handelsname Eumulgin® Prisma, Cognis GmbH).

### Tenside

In einer Ausführungsform der Erfindung enthalten die erfindungsgemäßen Zubereitungen als grenzflächen-aktive Verbindungen mindestens ein Tensid. Als grenzflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als **zwitterionische Tenside** werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die so genannten Betaine wie die N-Alkyl-N,N-dimethylammoniumglycinate, beispielsweise das Kokosalkyldimethyl-ammoniumglycinat, N-Acyl-aminopropyl-N,N-dimethyl-ammoniumglycinate, beispielsweise das Kokosacylaminopropyldimethylammoniumglycinat, und 2Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren (beispielsweise unter dem Handelsnamen Dehyton®DC kommerziell erhältlich), N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin. Weiterhin geeignten sind Derivate von N-Alkyliminodipropionsäuren, wie beispielsweise N-Lauryl-beta-Iminopropionate, kommerziell erhätlich unter dem Handelsnamen Deriphat® 160 C. Weiterhin geeignet sind Amphoacetate, wie z.B. Cocoamphoacetate (z.B. Dehyton® MC) oder Cocoamphodiacetate (wie z.B. Dehyton® DC).

**Anionische Tenside** sind gekennzeichnet durch eine wasserlöslich machende, anionische Gruppe wie z. B. eine Carboxylat-, Sulfat-, Sulfonat-, Citrat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze. Besonders geeignete anionische Tenside sind Glyceryl Stearate Citrate (wie z.B. kommerziell erhältlich unter den Handelsnamen Imwitor®370, Imwitor® 372P, Axol®C,62 or Dracorin®CE 614035) oder Glyceryl Stearat Lactat Verbindungen. Beispiel für ein geeignetes Alkylsulfat ist Sodium Cetearyl Sulfate (Handelsname Lanette® E), Beispiel für ein geeingetes Phosphate ist Potassium Cetyl Phosphate (Handelsname Amphisol® K). Beispiel für ein geeignetes Acylglutamat ist Sodium Stearoyl Glutamate (Handelsname z.B. Eumulgin® SG). Ein weiteres Beispiel für ein geeignetes anionische Tensid ist Sodium Lauryl Glucose Carboxylate (Handelsname Plantapon® LGC).

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Geeignete pseudo kationische Tenside sind beispielsweise Stearylaminopropyl Dimtheylamine (kommerziell erhältlich unter dem Handelsnamen Dehyquart® S18 oder Incromine® SB oderTegoAmide®S18). Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar. Geeignete kationische Tenside sind beispielsweise Dipalmitoylethyl Hydroxyethylmonium Methosulfate (Handelsname Dehyquart®C4046), Distearoylethyl Hydroxyethylmonium Methosulfate (Handelsname Dehyquart®F75), Dicocoylethyl Hydroxyethylmonium Methosulfate (Handelsname Dehyquart® L80), Behentrimonium Chloride (Handelsname Varisoft® BT), Distearyldimonium Chloride (Handelsname Varisoft® TA 100), Palmitamidopropyltrimonium Chloride (Handelsname Varisoft® PATC).

### Wachskomponente b-2)

In einer Ausführungsform der Erfindung enthalten die erfindungsgemäßen Zubereitungen mindestens eine Wachskomponente. Die erfindungsgemäßen Zubereitungen enthalten den/die Wachskomponente(n) in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zubereitung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30 C oder darüber schmelzen.
Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter, Shea Butter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten TriglyceridGemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C12-C18-Mono-, Di- und Triglyceriden) sowie Cutina® HVG (Hydrogenated Vegetable Glycerides) oder Cutina® GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der Cognis Deutschland GmbH unter der Bezeichnung Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zubereitungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Trüsostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar.

### Polymere b-3)

In einer Ausführungsform der Erfindung enthalten die erfindungsgemäßen Zubereitungen mindestens ein Polymer. Die erfindungsgemäßen Zubereitungen enthalten das/die Polymere(n) in einer Menge von 0 bis 20 Gew.-%, vorzugsweise 0,05 bis 18 Gew.-% vorzugsweise 0,05 bis 15 Gew.-%, besonders bevorzugt 0,05 bis 10 Gew.-%, insbesondere 0,1 bis 1 Gew.-% bezogen auf das Gesamtgewicht der Zubereitungen. In einer bevorzugten Ausführungsform der Erfindung enthalten die erfindungsgemäßen Zubereitungen das Polymer/die Polymere in einer Menge von 0,1 bis 5 Gew.-%, insbesondere 0,1 bis 3 Gew.-%, insbesondere 0,1 bis 2 Gew.-% bezogen auf das Gesamtgewicht der Zubereitung.

Geeignete **kationische Polymere** sind beispielsweise kationische Cellulosederivate, wie z. B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxy-propyl hydrolyzed collagen (Lamequat^{®}L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z. B. Mirapol^{®} A-15, Mirapol^{®} AD-1, Mirapol^{®} AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert. Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Besonders geeignete anionische Polymer sind solche mit der INCI Bezeichnung Carbomer, wie z.B die Carbopol Typen 980, 980,981,1382,2984,5984 sowie die unter den Handelsnamen Rheocare®C plus and Rheocare®400 erhältlichen Produkte. Weiterhin geeignete anionische Polymere sind solche mit dem INCI Namen Acrylates/C10-30 Alkyl Acrylate Crosspolymer (Handelsnamen z.B. Pemulen®TR , Pemulen® TR 2, Carbopol®Ultrez), Acrylates Copolymer (Handelsnamen z.B. Rheocare TTA, TTN, TTN-2), Acrylamide/Sodium Acrylate Copolymer (Handelsnamen z.B. Cosmedia®ATC), Sodium Polyacrylate (Handelsnamen z.B. Cosmedia® ATH, Cosmedia®SP), Polyacrylamides (Handelsnamen z.B. Sepigel® 305 or Sepigel® 501). Bevorzugte anionische Polymere sind Polyacrylsäure Homo- und Copolymere.

Weiterhin geeignete Polymere sind Silicone Elastomer Gums, wie z.B. Silikone Elastomer Gemische, wie z.B. Gemische mit den INCI Bezeichnungen Cycolpentasiloxane (and) Dimethiconol (and) Dimethicone Crosspolymer (Handelsname Dow Corning®DC 9027), Gemische mit der INCI Bezeichnung Isodecyl neopentanoate (and) Dimethicone / bisisobutyl PPG-20 Crosspolymer (Handelsname Dow Corning®DC EL 8051 IN), Gemische mit der INCI Bezeichnung Dimethicone / Vinyl Dimethicone Crosspolymer (and) C12-14 Pareth-12) (Handelsname Dow Corning®DC 9509) sowie Gemische mit der INCI Bezeichnung Dimethicone / Vinyl Dimethicone Crosspolymer (and) Silica (Handelsname Dow Corning®DC 9701 Cosmetic Powder).

Als Polymere eigen sich ebenso Polysaccharide, insbesondere Xanthan-Gum, Guar-Gum, Agar-Agar, Alginate und Tylosen sowie Tara Gum, Carraghenan, Sclerotium Gum und natürliche Cellulose.

### Weitere Ölkörper b-4)

Körperpflegemittel, wie Cremes, Körperöle, Lotionen und Milchen, enthalten üblicherweise eine Reihe weiterer Ölkörper und Emollients, die dazu beitragen, die sensorischen Eigenschaften weiter zu optimieren. Die Ölkörper (erfindungsgemäße Verbindungen plus weitere Ölkörper) sind üblicherweise in einer Gesamtmenge von 0,1 - 80, insbesondere 0,5 bis 70, bevorzugt 1 bis 60, insbesondere 1 bis 50 Gew.-%, insbesondere 1 bis 40 Gew.-%, vorzugsweise 5 - 25 Gew.-% und insbesondere 5 - 15 Gew.-% enthalten. Die weiteren Ölkörper sind üblicherweise in einer Menge von 0,1 bis 40 Gew.-% enthalten

Als weitere Ölkörper kommen beispielsweise Guerbetalkohole auf Basis von Fettalkoholen mit 6 bis 18, vorzugsweise 8 bis 10 Kohlenstoffatomen, in Frage sowie Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen und Kohlenwasserstoffen oder deren Gemischen. Weiterhin geeignet sind Ester von 2-Propylheptanol mit n-Octansäure, wie z.B. kommerziell erhältlich unter dem Handelsnamen Cetiol®SenSoft (Cognis GmbH). Weiterhin geeignet sind Kohlenwasserstoffe, wie zum Beispiel Undecan und Tridecan. Weiterhin geeignet sind Alkane, wie z.B. die Gemische mit der INCI Bezeichnung Conocnut/Palm/Palm Kernel Oil Alkanes (Handelsname Vegelight 1214 der Fa. Biosynthesis).

Überraschenderweise wurde gefunden, dass sich die erfindungsgemäßen Verbindungen insbesondere eignen um öllösliche kristalline UV-Lichtschutzfilter zu solubilisieren.

Ein Gegenstand der Erfindung betrifft Zubereitungen, enthaltend wenigstens eine Verbindung gemäß Anspruch 1 und mindestens einen UV-Lichtschutzfilter, vorzugsweise einen öllöslichen UV-Lichtschutzfilter.

Erfindungsgemäß sind als UV-Lichtschutzfilter bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
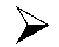 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
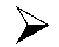 3-(4'-Trimethylammonium) benzyliden- bornan-2-on-methylsulfat (Mexoryl SO)
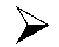 3,3'-(1,4-Phenylendimethin)-bis (7,7-dimethyl-2-oxobicyclo-[2.2.1]heptan-1-methansulfonsäure) and salts (Mexoryl SX)
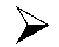 3-(4'-Sulfo)-benzyliden-bornan-2-on and salts (Mexoryl SL)
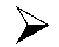 Polymer von N-{(2und 4)-[2-oxoborn-3-yliden)methyl}benzyl]acrylamid (Mexoryl SW)
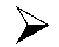 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl SL)
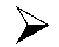 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoe-säureamylester;
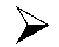 Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
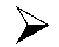 Ester der Salicylsäure, vorzugsweise Salicylsäure-2-ethylhexylester, Salicylsäure-4-iso-propylbenzylester, Salicylsäurehomomenthylester;
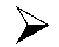 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
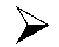 Ester der Benzalmalonsäure, vorzugsweise 4-Methoxybenzmalonsäuredi-2-ethylhexylester;
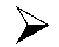 Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl)anilino]-1,3,5-triazin (Uvinul T 150) wie in der EP 0818450 A1 beschrieben oder 4,4'-[(6-[4-((1,1-Dimethylethyl)amino-carbonyl)phenyl-amino]-1,3,5-triazin-2,4- diyl)diimino]bis(benzoesäure-2-ethylhexylester) (Uvasorb® HEB);
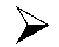 2,2(-Methylen-bis(6-(2H-benzotriazol-2-yl)-4-(1,1,3,3-tetramethyl-butyl)phenol) (Tinosorb M);
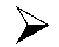 2,4-Bis[4-(2-ethylhexyloxy)-2-hydroxyphenyl]-6-(4-methoxyphenyl)-1,3,5-triazin (Tinosorb S);
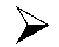 Propan-1,3-dione, wie z.B. 1-(4-tert.Butylphenyl)-3-(4'methoxyphenyl)propan-1,3-dion;
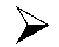 Ketotricyclo(5.2.1.0)decan-Derivate, wie in der EP 0694521 B1 beschrieben;
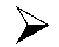 Dimethicodiethylbenzalmalonate (Parsol SLX).

Als wasserlösliche UV - Filter kommen in Frage:
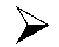 2-Phenylbenzimidazol-5-sulfonsäure und deren Alkali-, Erdalkali-, Ammonium-, Alkylammonium-, Alkanolammonium- und Glucammoniumsalze;
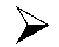 2,2(-(1,4-Phenylen)bis(1H-benzimidazol-4,6-disulfon-säure, Mononatriumsalz) (Neo Heliopan AP)
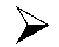 Sulfonsäurederivate von Benzophenonen, vorzugsweise 2-Hydroxy-4-methoxybenzo-phenon-5-sulfonsäure und ihre Salze;
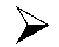 Sulfonsäurederivate des 3-Benzylidencamphers, wie z.B. 4-(2-Oxo-3-bornylidenmethyl)-benzolsulfonsäure und 2-Methyl-5-(2-oxo-3-bornyliden)sulfonsäure und deren Salze.
In einer bevorzugten Ausführungsform der Erfindung enthalten die Zubereitungen mindestens einen öllöslichen UV-Lichtschutzfilter sowie mindestens einen wasserlöslichen UV-Lichtschutzfilter.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).
Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Die erfindungsgemäßen Zubereitungen können auch unlösliche Lichtschutzpigmente, nämlich feindisperse Metalloxide bzw. Salze enthalten. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

In einer bevorzugten Ausführungsform der Erfindung enthalten die Zubereitungen mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden-bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzyliden-bornan-2-on und ihre Salze, Polymer von N-{(2und 4)- [2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy)disiloxanyl)propyl)phenol, Dimethicodiethyl benzalmalonate und ihren Mischungen.
Diese UV-Lichtschutzfilter sind beispielsweise unter den folgenden Handelsnamen kommerziell erhältlich:
NeoHeliopan®MBC (INCI: 4-Methylbenzylidene Camphor; Hersteller: Symrise); NeoHeliopan® BB (INCI: Benzophenone-3, Hersteller: Symrise); Parsol®1789 (INCI: Butyl Methoxydibenzoylmethane, Hersteller: Hoffmann-La Roche (Givaudan); Tinosorb®S (INCI: Bis-Ethylhexyloxyphenol Methoxyphenyl Triazine); Tinosorb®M (INCI: Methylene Bis-Benzotriazolyl Tetramethylbutylphenol): Herstelller: Ciba Specialty Chemicals Corporation; Uvasorb®HEB (INCI: Diethylhexyl Butamido Triazone, Hersteller: 3V Inc.), Uvinul®T 150 (INCI: Ethylhexyl Triazone, Hersteller: BASF AG); Uvinul® A plus (INCI: Diethylamino Hydroxybenzoyl Hexyl Benzoate: Hersteller: BASF AG; Mexoryl® SO: 3-(4'-Trimethylammonium) benzyliden- bornan-2-on-methylsulfat, INCI: Camphor Benzalkonium Methosulfate; Mexoryl®SX: 3,3'-(1,4-Phenylendimethin)-bis (7,7- dimethyl-2-oxobicyclo-[2.2.1] heptan-1-methansulfonsäure), CTFA: INCI Terephthalylidene Dicamphor Sulfonic Acid; Mexory® SL: 3-(4'-Sulfo)-benzyliden-bornan-2-on, INCI Benzylidene Camphor Sulfonic Acid; Mexoryl®SW: Polymer von N-{(2und 4)-[2-oxoborn-3-yliden)methyl}benzyl]acrylamid, INCI Polyacrylamidomethyl Benzylidene Camphor; Mexoryl®SL: 2-(2H-Benzotriazol-2-yl)-4-methyl-6-(2-methyl-3-(1,3,3,3-tetramethyl-1-(trimethylsilyloxy)disiloxanyl)propyl) phenol; INCI: DROMETRIZOLE TRISILOXANE; Parsol® SLX: Dimethicodiethylbenzalmalonate, INCI Polysilicone-15.

Die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen auf die Zubereitung - enthalten.

### Weitere Inhaltsstoffe

Als **Verdickungsmittel** eignen sich beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox). Ein geeigneter Verdicker ist beispielsweise das unter den Handelsnamen Cosmedia® Gel CC erhältliche Produkt mit der INCI Bezeichnung Dicaprylyl Carbonate, Stearalkonium Hectorite and Propylene Carbonate. Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen. **Desodorierende Wirkstoffe / Antiperspirantien** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker. Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage. Als **Selbstbräuner** eignet sich Dihydroxyaceton oder Erythrulose. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage. Als **Konservierungsmittel** eignen sich beispielsweise Phenoxyethanol, Formaldehydlösung, Parabene, Pentandiol, Chlorphenesin, Caprylylglykol, Ethylhexylglycerine oder Sorbinsäure sowie die unter der Bezeichnung Surfacine® bekannten Silberkomplexe und die in Anlage 6, Teil A und B der Kosmetikverordnung aufgeführten weiteren Stoffklassen. Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage. Als **Perlglanzwachse oder Perlglanz-Verbindungen,** insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Stearyl Citrate, Cyclodextrin, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen. Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Ein geeignetes Überfettungsmittel ist beispielsweise die Mischung von Cocoglucosiden und Glyceryl Oleate (kommerziell erhältlich als Lamesoft® PO65 von Cognis GmbH).
Geeignete Füllstoffe sind Substanzen, die beispielsweise die sensorischen oder kosmetischen Eigenschaften einer Zubereitung verbessern und die beispielsweise ein samtiges oder seidiges Gefühl erzeugen oder verstärken (sog. Skin-Sensory Modifier). Geeignete Füllstoffe sind Stärke und Stärkederivate (wie z.B. Tapioka Stärke, Aluminium Starch Octenyl Succinate, Sodium Octenyl Succinat, Distärke Phosphat), Pigmente, die nicht hauptsächlich als UV-Filter oder Farbstoffe dienen (wie z.B. Bornitrid) und/oder Aerosil^{®} (CAS-Nr. 7631-86-9), und/oder Talkum, sowie beispielsweise Polymethyl Methacrylate (z.B. Cosmedia^{®} PMMA V8/V12), Silica (z. B. Cosmedia^{®} SILC), Stearalkonium Hectorite (wie im kommerziell erhältlichen Produkt Cosmedia® Gel CC enthalten) sowie HDI/Trimethylol Hexyllactone Crosspolymer (wie im kommerziell erhältlichen Produkt Cosmedia^{®} CUSHION enthalten).

Als **Stabilisatoren** können Metallsalze von Fettsäuren, wie z. B. Magnesium-, Aluminium-und/oder Zinkstearat bzw. -ricinoleat eingesetzt werden. Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Die erfindungsgemäßen Zubereitungen, sowie die Verbindung gemäß Anspruch 1 eignen sich insbesondere in kosmetischen und/oder pharmazeutischen Zubereitungen zur Benetzung oder Imprägnierung oder Beschichtung von Gebrauchs- und Hygienetüchern, die zur Körperreinigung und/oder zur Körperpflege eingesetzt werden.

Als Gebrauchs- und Hygienetücher seien exemplarisch genannt: Tissues, Papiere, Wipes, Vlies-Produkte, Schwämme, Puffs, Pflaster und Bandagen, die ihren Einsatz im Bereich der Hygiene und Pflege finden. Dies können sein Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte sowie Selbstbräunungs-Wipes.

### Beispiele

### (1) Herstellbeispiele

### Synthese der Mischether im Labor

Nachfolgende Synthesen wurden mit folgenden Spezifikationen/ Handelsprodukten durchgeführt:
Lorol® - "Lorol technisch", C12-18 Fettalkohol mit folgender Kettenverteilung:

| | |
|---|---|
| C12 | 48 - 58 % |
| C14 | 18 - 24 % |
| C16 | 8 - 12 % |
| C18 | 11 - 15% |

Lorol C 8 : 1-Octanol, mindestens 95 % C8-Fettalkohol
Lorol C10 : 1-Decanol, mindestens 94 % C10-Fettalkohol
Behenylalkohol (Stenol® 1822) n-Docosanol mit mindestens 42 % C22-Fettalkohol.
Isostearylalkohol (Prisorine® 3515)

### Eingesetzte Apparatur:

1 L 4-Halsrührapparatur, N2-Einleitung, Wasserabscheider

### A) Mischether - C12-C18 - Fettalkohol / Isostearylalkohol

**Ansatz: A (75/25)**

| | | |
|---|---|---|
| I | 225,0 g | Lorol® techn. (C12-C18 - Fettalkohole) |
| II | 75,0 g | Prisorine® 3515; Unichema (Isostearylalkohol, C18-OH) |
| III | 0,6 g | CF3SO3H (50%ig) = 2 mmol (0,1 % AS auf I und II) |
| IV | 1,5 g | H3PO2 (50%ig) = 11,4 mmol |
| V | 2,0 g | NaOH (50%ig) = 25 mmol |

### Durchführung:

Die Ausgangsstoffe I-IV wurden vorgelegt, mit Stickstoff begast und 1,5 Stunden bei 220°C gerührt. Das resultierende Reaktionsprodukt wurde dann mit Natronlauge (V) bei 80°C neutralisiert. Um das entstandene Olefin und den nicht umgesetzten Fettalkohol zu entfernen, wurde das Reaktionsgemisch in einer Destillationsapparatur bis zu einer Sumpftemperatur von 200°C/<0,1 mbar andestilliert.
Zur Aufreinigung und Entfernung der Katalysatorsalze wurde das Zwischenprodukt dann 2 Stunden bei 90°C mit 2 % Tonsil Standard (6,0 g; Filtrierhilfsmittel) verrührt und heiß über eine Glasfilternutsche abgesaugt.

### B) Mischether - C12-C18 - Fettalkohol / Isostearylalkohol

**Ansatz: B (90/10)**

| | | |
|---|---|---|
| I | 450,0 g | Lorol® techn. (C12-C18 - Fettalkohole) |
| I | 50,0 g | Prisorine® 3515; Unichema (Isostearylalkohol, C18-OH) |
| III | 0,5 g | CF3SO3H (50%ig) = 1,7 mmol |
| IV | 2,5 g | H3PO2 (50%ig) = 18,9 mmol |
| V | 3,17 g | NaOH (50%ig) = 39,6 mmol |

### Durchführung:

Die Ausgangsstoffe I-IV wurden vorgelegt, mit Stickstoff begast und 4 Stunden bei 220°C gerührt. Das resultierende Reaktionsprodukt wurde mit Natronlauge (V) bei 50°C neutralisiert und das Gemisch bis zu einer Sumpftemperatur von 200°C/<0,1 mbar andestilliert.
Zur Aufreinigung wurde das Zwischenprodukt dann 2 Stunden bei 90°C mit 2 % Tonsil Standard (10,0 g) verrührt und warm über eine Glasfilternutsche abgesaugt.

### C) Mischether - C12-C18 - Fettalkohol / Behenylalkohol/ Isostearylalkohol

**Ansatz: C (33,3/33,3/33,3)**

| | | |
|---|---|---|
| I | 160,0 g | Lorol® techn. (C12-C18 - Fettalkohole) |
| I | 160,0 g | Stenol® 1822 SR (Behenylalkohol) |
| III | 160,0 g | Prisorine® 3515; Unichema (Isostearylalkohol, C18-OH) |
| IV | 0,48 g | CF₃SO₃H (50%ig) = 1,6 mmol |
| V | 2,4 g | H₃PO₂ (50%ig) = 21,2 mmol |
| VI | 3,04 g | NaOH (50%ig) = 38 mmol |

### Durchführung:

Die Ausgangsstoffe I-V wurden vorgelegt, mit Stickstoff begast und 4 Stunden bei 220°C gerührt. Das resultierende Reaktionsprodukt wurde mit Natronlauge (VI) bei 80°C neutralisiert und das Gemisch bis zu einer Sumpftemperatur von 200°C/<0,1mbar andestilliert.
Zur Aufreinigung wurde das Zwischenprodukt dann 2 Stunden bei 90°C mit 2 % Tonsil Standard (9,6 g) verrührt und warm über eine Glasfilternutsche abgesaugt.

### D) Mischether - C12-C18 - Fettalkohol / Behenylalkohol/ Isostearylalkohol

**Ansatz: D (25/25/50)**

| | | |
|---|---|---|
| I | 125,0 g | Lorol® techn. (C12-C18 - Fettalkohole) |
| II | 125,0 g | Stenol® 1822 SR (Behenylalkohol) |
| III | 250,0 g | Prisorine® 3515; Unichema (Isostearylalkohol, C18-OH) |
| IV | 0,50 g | CF₃SO₃H (50%ig) = 1,7 mmol (0,05% auf I,II und III) |
| V | 2,5 g | H₃PO₂ (50%ig) = 18,9 mmol |
| VI | 3,16 g | NaOH (50%ig) = 39,5 mmol |

### Durchführung:

Die Ausgangsstoffe I-V wurden vorgelegt, mit Stickstoff begast und 4 Stunden bei 220°C gerührt. Das resultierende Reaktionsprodukt wurde mit Natronlauge (VI) bei 80°C neutralisiert und das Gemisch bis zu einer Sumpftemp. von 200°C/<0,1 mbar andestilliert. Abdestilliert wurden 20,1 g und 461,0 g Zwischenprodukt wurden gewonnen.
Zur Aufreinigung wurde das Zwischenprodukt dann 2 Stunden bei 90°C mit 2 % Tonsil Standard (10,0 g) verrührt und heiß über eine Glasfilternutsche abgesaugt.
Die Analytik des Reaktionsproduktes ergab einen Reinhaltsgehalt von ca. 96% GC (CT-CP).

### E) Mischether - C12-C18 - Fettalkohol / Behenylalkohol/ Isostearylalkohol

**Ansatz: E (50/16,7/33,3)**

| | | |
|---|---|---|
| I | 225,0 g | Lorol® techn. (C12-C18 - Fettalkohole) |
| I | 75,0 g | Stenol® 1822 SR (Behenylalkohol) |
| III | 150,0 g | Prisorine® 3515; Unichema (Isostearylalkohol, C18-OH) |
| IV | 0,45 g | CF₃SO₃H (50%ig) = 1,5 mmol |
| V | 2,25 g | H₃PO₂ (50%ig) = 17 mmol |
| VI | 2,85 g | NaOH (50%ig) = 35,6 mmol |

### Durchführung:

Die Ausgangsstoffe I-V wurden vorgelegt, mit Stickstoff begast und 4 Stunden bei 220°C gerührt. Das resultierende Reaktionsprodukt wurde mit Natronlauge (VI) bei 80°C neutralisiert und das Gemisch bis zu einer Sumpftemp. von 200°C/<0,1mbar andestilliert. Zur Aufreinigung wurde das Zwischenprodukt dann 2 Stunden bei 90°C mit 2 % Tonsil Standard (9,0 g) verrührt und heiß über eine Glasfilternutsche abgesaugt.

### F) Mischether - C12-C18 - Fettalkohol / Behenylalkohol/ Isostearylalkohol

**Ansatz: F (70/5/25)**

| | | |
|---|---|---|
| **I** | 280,0 g | Lorol® techn. (C12-C18 - Fettalkohole) |
| II | 20,0 g | Stenol® 1822 SR (Behenylalkohol) |
| III | 100,0 g | Prisorine® 3515; Unichema (Isostearylalkohol, C18-OH) |
| IV | 0,4 g | CF₃SO₃H (50%ig) = 0,05 mmol |
| V | 2,0 g | H₃PO₂ (50%ig) = 15,2 mmol |
| VI | 2,52 g | NaOH (50%ig) = 31,6 mmol |

### Durchführung:

Die Ausgangsstoffe I-V wurden vorgelegt, mit Stickstoff begast und 4 Stunden bei 220°C gerührt. Das resultierende Reaktionsprodukt wurde mit Natronlauge (VI) bei 80°C neutralisiert und das Gemisch bis zu einer Sumpftemp. von 200°C/<0,1 mbar andestilliert. Zur Aufreinigung wurde das Zwischenprodukt dann 2 Stunden bei 90°C mit 2 % Tonsil Standard (8,0 g) verrührt und heiß über eine Glasfilternutsche abgesaugt.

### G) Mischether - C12-C18, C8, C10 - Fettalkohol / Behenylalkohol/ Isostearyl-alkohol

**Ansatz: G (25/25/25/12,5/12,5)**

| | | |
|---|---|---|
| I | 50,0 g | Lorol® C8 (1-Octanol) |
| II | 50,0 g | Lorol® C10 (1-Decanol) |
| III | 100,0g | Lorol® techn. (C12 - C18 Fettalkohole) |
| IV | 100,0g | Stenol® 1822 SR (Behenylalkohol) |
| V | 100,0g | iso-C18-OH (Prisorine® 3515; Unichema) Isostearylalkohol |
| VI | 0,4g | CF₃SO₃H (50%ig) = 1,3mmol |
| VII | 2,0g | H₃PO₂ (50%ig) = 15,2mmol |
| VII | 2,52g | NaOH (50%ig) = 31,6mmol |

### Durchführung:

Die Ausgangsstoffe I-VIII wurden vorgelegt, mit Stickstoff begast und 4 Stunden bei 220°C gerührt. Das resultierende Reaktionsprodukt wurde mit Natronlauge (VIII) bei 80°C neutralisiert und das Gemisch bis zu einer Sumpftemp. von 150°C und einer Brüdentemperatur von 94°C (gleichmäßiger Dest.-Anfall)/<0,1mbar andestilliert. Abdestilliert wurden 11,8 g und 365,5 g Zwischenprodukt wurden gewonnen.

Zur Aufreinigung wurde das Zwischenprodukt dann 2 Stunden bei 90°C mit 2 % Tonsil Standard (8,0 g) verrührt und heiß über eine Glasfilternutsche abgesaugt.

Die Analytik des Reaktionsproduktes ergab einen Reinhaltsgehalt von ca. 96,5 % GC (CT-CP).

### (2) Formulierung und Sensorik

Die Anwendungseigenschaften der Mischether wurden in einer kosmetischen Formulierung mit 1% Cosmedia® SP (Natriumpolyacrylat), 10 % Cetiol® LC (Coco-Caprylat/Caprat) und 3 % Glycerin untersucht. Die Einsatzkonzentration an Mischethern bzw. Vaseline betrug jeweils 6%.

**Tabelle 2.1 - Formulierung:**

| | **F-V** | **F-D** | **F-G** |
|---|---|---|---|
| | | | |
| COSMEDIA® SP (Natriumpolyacrylat) | 1,0 | 1,0 | 1,0 |
| CETIOL® LC (Coc-Caprylat / Caprat) | 10,0 | 10,0 | 10,0 |
| Vaseline, weiss (Sigma Aldrich) | **6,0** | -- | -- |
| Mischether Ansatz D | -- | **6,0** | -- |
| Mischether Ansatz G | | | **6,0** |
| Glycerol | 3,0 | 3,0 | 3,0 |
| Wasser, demin. | 79,9 | 79,9 | 79,9 |
| Euxyl K 100 (Konservierungsmittel) | 0,1 | 0,1 | 0,1 |
| pH-Wert | 6,1 | 6,0 | 6,0 |
| Viskosität (Brookfield, RVF, 23°C, Spindel TE, 4 UpM, mit Helipath) mPa*s | 162500 | 137500 | 125000 |

### Sensorik im Vergleich zu Vaseline in einer kosmetischen Formulierung:

Die Sensorik der Formulierung wurde von 5 Testpersonen nach definierten Kriterien bewertet. Vaseline stellt den Standard im Vergleich dar (+ beschreibt das Urteil einer Testperson).
Das Muster mit Mischether nach Ansatz D (Formulierung F-D) zeigt vergleichbare sensorische Eigenschaften wie die Vaseline-Formulierung. Insbesondere zieht es im Vergleich zum Standard deutlich schneller in die Haut ein und wird als etwas trockener empfunden.

**Tabelle 2.2 - Sensorischer Test (n = 5)**

| | **-** | **Standard** | + | |
|---|---|---|---|---|
| Spreitung (gering) | + | +++ | + | Spreitung (hoch) |
| Absorption 1min (langsam) | | ++ | +++ | Absorption 1 min (schnell) |
| Absorption 3min (langsam) | | + | ++++ | Absorption 3 min (schnell) |
| Rückstände (viel) | | +++ | ++ | Rückstände (wenig) |
| Klebrigkeit (stark) | | ++++ | + | Klebrigkeit (gering) |
| Öligkeit (stark) | ++ | ++ | + | Öligkeit (gering) |
| Wachsartigkeit (stark) | + | ++ | ++ | Wachsartigkeit (gering) |
| Samtigkeit (gering) | | +++++ | | Samtigkeit (ausgeprägt) |
| Seidigkeit (gering) | | +++++ | | Seidigkeit (ausgeprägt) |
| Pulvergefühl (gering) | | +++++ | | Pulvergefühl (ausgeprägt) |
| Weichheit (gering) | ++ | ++ | + | Weichheit (ausgeprägt) |
| Glätte (gering) | ++ | ++ | + | Glätte (ausgeprägt) |
| Pflegegefühl (gering) | ++ | +++ | | Pflegegefühl (ausgeprägt) |
| Akzeptanz (gering) | | ++++ | + | Akzeptanz (hoch) |

### (3) Einsatz in Tensidsystemen

Die Anwendungseigenschaften der Mischether wurden in einer tensidischen Formulierung mit 16,1 % Texapon® N70 (Natriumlaurethsulfat 2EO), 11,1 % Dehyton® PK45 (Cocamidopropylbetain), 2,15 % Comperlan® CMEA (Cocamide MEA), 4% Sonnenblumenöl, 4% Edenor® C12 (Laurinsäure), 0,2 % Dehyquart® GUAR N (Guarhydroxypropyltrimoniumchlorid), 0,2 % EDTA BD, 0,5 % Glycerin, 0,5 % NatriumBenzoat und 1,2 % Zitronensäure untersucht. Die Einsatzkonzentration an Mischethern bzw. Vaseline betrug jeweils 4 Gew. %.

**3.1 Rezeptur: Testformulierung für Tensidsystem**

| | **T-D** | **T-G** | **T-V Vergleich** |
|---|---|---|---|
| TEXAPON® N70 | 16,1 | 16,1 | 16,1 |
| DEHYTONL® PK45 | 11,1 | 11,1 | 11,1 |
| COMPERLAN® CMEA | 2,15 | 2,15 | 2,15 |
| Sonnenblumenöl | 4,0 | 4,0 | 4,0 |
| Edenor® C 12 | 4,0 | 4,0 | 4,0 |
| Vaseline, weiss (Sigma Aldrich) | -- | - | **4,0** |
| Mischether Ansatz D | **4,0** | -- | -- |
| Mischether Ansatz G | | **4,0** | |
| DEHYQUART® GUAR N | 0,2 | 0,2 | 0,2 |
| EDTA BD | 0,2 | 0,2 | 0,2 |
| Glycerol | 0,5 | 0,5 | 0,5 |
| Natriumbenzoat | 0,5 | 0,5 | 0,5 |
| Wasser, demin. | 56,05 | 56,55 | 56,55 |
| Zitronensäure (50%) | 0,75 | 0,8 | 0,7 |
| pH-Wert | 4,9 | 4,9 | 4,9 |
| Viskosität (Brookfield, RVF, 23°C, Spindel 5, 10 UpM) mPa*s | 15200 | 15400 | 14400 |

### Schäumungseigenschaften

Die Schäumungseigenschaften wurden in einem Sita Rotorfoam Messgerät in einer 1%igen Lösung bei 15°dH bei 30°C bestimmt. Das Anschäumverhalten der beiden Mischether-Formulierungen nach 30 Sekunden ist vergleichbar zur Formulierung mit Vaseline. Im weiteren Verlauf steigt die Schaummenge bei den Mischether-Formulierungen leicht an, während die Schaummenge der Vaseline-Formulierung konstant bleibt.

**Tab. 3.2: Schäumungseigenschaften**

| | Schaumhöhe in ml | | |
|---|---|---|---|
| Zeit [s] | **T-V** | **T-D** | **T-G** |
| 30 | 126 | 134 | 144 |
| 60 | 134 | 180 | 163 |
| 90 | 139 | 210 | 181 |
| 120 | 150 | 249 | 197 |
| 150 | 154 | 259 | 207 |
| 180 | 156 | 286 | 219 |
| 210 | 149 | 301 | 229 |
| 240 | 150 | 314 | 229 |
| 270 | 150 | 335 | 236 |
| 300 | 126 | 346 | 238 |

### (4) Okklusivität

Über eine Bestimmung des TEWL (Transepidermaler Wasserverlust) wurde die Okklusivität der Mischether bestimmt.

Die Okklusivwirkung wurde über die Reduktion der Wasserdurchlässigkeit der Haut mit Hilfe eines Evaporimeter-Verfahrens bestimmt. Dazu wurde der Wasserdampfgradient über der ölbehandelten bzw. unbehandelten Haut des Unterarms mit zwei Messsonden in klimatisierter Umgebung gemessen und daraus die Wasserdurchlässigkeit der Haut ermittelt.

Vaseline wurde als Positiv-Standard, IPM als Negativ-Standard verwendet. Im Folgenden die Einordnung der Proben hinsichtlich der Okklusivität:

| | |
|---|---|
| 1. Vaseline | - ausgeprägt bis stark okklusiv |
| 2. Mischether Ansatz D | - mäßig bis stark okklusiv |
| 3. Mischether Ansatz G | - mäßig bis stark okklusiv |
| 4. IPM - CE92010016 | - ausgeprägt bis wenig okklusiv |

Beide untersuchten Mischether zeigen stark okklusive Eigenschaften und sind somit gut als Vaseline-Ersatz geeignet.

## Patentansprüche

1. Mischether, erhältlich durch Kondensationsreaktion von
a) 25 bis 90 Gew.% von unverzweigten Fettalkoholen mit einer Kettenlänge von 12 bis 18 Kohlenstoffatomen
b) 5 bis 50 Gew. % Isostearylalkohol
c) 0 bis 35 Gew. % eines Fettalkohols mit einer Kettenlänge von 20 und/oder 22 Kohlenstoffatomen und
d) 0 bis 25 Gew. % eines Fettalkohols mit 8 und/oder 10 Kohlenstoffatomen **dadurch gekennzeichnet, dass** sie einen nach dynamischer Differenzkalorimetrie (DSC) gemessenen Schmelzbereich zwischen -25 °C und +70 °C aufweisen, wobei die Breite des Schmelzbereichs mindestens 30 Temperaturgrade umfasst und das Maximum des Schmelzbereichs bei 35 ± 10 °C liegt.

2. Mischether nach Anspruch 1, erhältlich durch Kondensationsreaktion von
a) 25 bis 70 Gew.% von unverzweigten Fettalkoholen mit einer Kettenlänge von 12 bis 18 Kohlenstoffatomen und
b) 5 bis 50 Gew. % Isostearylalkohol
c) 1 bis 35 Gew. % eines Fettalkohols mit einer Kettenlänge von 20 und/oder 22 Kohlenstoffatomen und
d) 0 bis 25 Gew. % eines Fettalkohols mit 8 und/oder 10 Kohlenstoffatomen.

3. Mischether nach Anspruch 1, erhältlich durch Kondensationsreaktion von
a) 25 bis 50 Gew.% von unverzweigten Fettalkoholen mit einer Kettenlänge von 12 bis 18 Kohlenstoffatomen
b) 25 bis 50 Gew. % Isostearylalkohol
c) 15 bis 35 Gew. % eines Fettalkohols mit einer Kettenlänge von 20 und/oder 22 Kohlenstoffatomen und
d) gegebenenfalls 10 bis 25 Gew. % eines Fettalkohols mit 8 und/oder 10 Kohlenstoffatomen.

4. Mischether nach Anspruch 1, erhältlich durch Kondensationsreaktion von
a) 25 bis 35 Gew.% von unverzweigten Fettalkoholen mit einer Kettenlänge von 12 bis 18 Kohlenstoffatomen und
b) 33 bis 50 Gew. % Isostearylalkohol und
25 bis 35 Gew. % eines Fettalkohols mit einer Kettenlänge von 20 und/oder 22 Kohlenstoffatomen.

5. Mischether gemäß den Ansprüchen 1 bis 4, **dadurch gekennzeichnet, dass** sie einen Schmelzbereich zwischen -22 °C und +55 °C aufweisen, wobei die Breite des Schmelzbereichs mindestens 50 Temperaturgrade umfasst und das Maximum des Schmelzbereichs bei 35 ± 10 °C liegt.

6. Mischether gemäß den Ansprüchen 1 bis 5, **dadurch gekennzeichnet, dass** die Komponente a) aus unverzweigten, gesättigten Fettalkoholen mit folgender Kettenverteilung:
C12 von 48 - 58 Gew. %
C14 von 18 - 24 Gew. %
C16 von 8 - 12 Gew. %
C18 von 11 - 15 Gew. % besteht.

7. Mischether gemäß den Ansprüchen 1 bis 6, **dadurch gekennzeichnet, dass** die Komponente c) n-Docosanol ist.

8. Mischether gemäß den Ansprüchen 1 bis 5, **dadurch gekennzeichnet, dass** die Kondensationsreaktion bei Temperaturen von 200 bis 250 °C durchgeführt wird.

9. Mischether gemäß den Ansprüchen 1 bis 5, **dadurch gekennzeichnet, dass** bei der Kondensationsreaktion Sulfonsäure und/oder Methansulfonsäure gegebenenfalls in Kombination mit Phosphorsäuren eingesetzt werden.

10. Kosmetische und/oder pharmazeutische Zubereitungen enthaltend Mischether gemäß einem der Ansprüche 1 bis 9.

11. Verwendung von Mischethern gemäß einem der Ansprüche 1 bis 9 als Ersatz von Vaseline in kosmetischen oder pharmazeutischen Zubereitungen.

## Claims

1. A mixed ether obtainable by condensation reaction of
a) 25 to 90% by weight of unbranched fatty alcohols with a chain length of 12 to 18 carbon atoms
b) 5 to 50% by weight of isostearyl alcohol
c) 0 to 35% by weight of a fatty alcohol with a chain length of 20 and/or 22 carbon atoms and
d) 0 to 25% by weight of a fatty alcohol having 8 and/or 10 carbon atoms
wherein it has a melting range, measured by differential scanning calorimetry (DSC), between - 25°C and +70°C, where the width of the melting range comprises at least 30 temperature degrees and the maximum of the melting range is 35 ± 10°C.

2. A mixed ether according to claim 1, obtainable by condensation reaction of
a) 25 to 70% by weight of unbranched fatty alcohols with a chain length of 12 to 18 carbon atoms and
b) 5 to 50% by weight of isostearyl alcohol,
c) 1 to 35% by weight of a fatty alcohol with a chain length of 20 and/or 22 carbon atoms and
d) 0 to 25% by weight of a fatty alcohol having 8 and/or 10 carbon atoms.

3. A mixed ether according to claim 1, obtainable by condensation reaction of
a) 25 to 50% by weight of unbranched fatty alcohols with a chain length of 12 to 18 carbon atoms
b) 25 to 50% by weight of isostearyl alcohol and
c) 15 to 35% by weight of a fatty alcohol with a chain length of 20 and/or 22 carbon atoms and
d) optionally 10 to 25% by weight of a fatty alcohol having 8 and/or 10 carbon atoms.

4. A mixed ether according to claim 1, obtainable by condensation reaction of
a) 25 to 35% by weight of unbranched fatty alcohols with a chain length of 12 to 18 carbon atoms and
b) 33 to 50% by weight of isostearyl alcohol and 25 to 35% by weight of a fatty alcohol with a chain length of 20 and/or 22 carbon atoms.

5. The mixed ether according to claims 1 to 4, wherein it has a melting range between -22°C and +55°C, where the width of the melting range comprises at least 50 temperature degrees and the maximum of the melting range is 35 ± 10°C.

6. The mixed ether according to claims 1 to 5, wherein the component a) consists of unbranched, saturated fatty alcohols having the following chain distribution:
C12 from 48-58% by weight
C14 from 18-24% by weight
C16 from 8-12% by weight
C18 from 11-15% by weight.

7. The mixed ether according to claims 1 to 6, wherein component c) is n-docosanol.

8. The mixed ether according to claims 1 to 5, wherein the condensation reaction is carried out at temperatures of from 200 to 250°C.

9. The mixed ether according to claims 1 to 5, wherein sulfonic acid and/or methanesulfonic acid, optionally in combination with phosphoric acids, are used in the condensation reaction.

10. A cosmetic and/or pharmaceutical preparation comprising mixed ethers according to any one of claims 1 to 9.

11. The use of mixed ethers according to any one of claims 1 to 9 as a replacement for Vaseline in cosmetic or pharmaceutical preparations.

## Revendications

1. Éthers mixtes, pouvant être obtenus par une réaction de condensation de
a) 25 à 90 % en poids d'alcools gras non ramifiés ayant une longueur de chaîne de 12 à 18 atomes de carbone,
b) 5 à 50 % en poids d'alcool isostéarylique,
c) 0 à 35 % en poids d'un alcool gras ayant une longueur de chaîne de 20 et/ou 22 atomes de carbone, et
d) 0 à 25 % en poids d'un alcool gras contenant 8 et/ou 10 atomes de carbone,
**caractérisés en ce qu'**ils présentent une plage de fusion mesurée par calorimétrie différentielle dynamique (DSC) comprise entre -25 °C et +70 °C, la largeur de la plage de fusion comprenant au moins 30 degrés de température et le maximum de la plage de fusion se situant à 35 ± 10 °C.

2. Éthers mixtes selon la revendication 1, pouvant être obtenus par une réaction de condensation de
a) 25 à 70 % en poids d'alcools gras non ramifiés ayant une longueur de chaîne de 12 à 18 atomes de carbone, et
b) 5 à 50 % en poids d'alcool isostéarylique,
c) 1 à 35 % en poids d'un alcool gras ayant une longueur de chaîne de 20 et/ou 22 atomes de carbone, et
d) 0 à 25 % en poids d'un alcool gras contenant 8 et/ou 10 atomes de carbone.

3. Éthers mixtes selon la revendication 1, pouvant être obtenus par une réaction de condensation de
a) 25 à 50 % en poids d'alcools gras non ramifiés ayant une longueur de chaîne de 12 à 18 atomes de carbone,
b) 25 à 50 % en poids d'alcool isostéarylique,
c) 15 à 35 % en poids d'un alcool gras ayant une longueur de chaîne de 20 et/ou 22 atomes de carbone, et
d) éventuellement 10 à 25 % en poids d'un alcool gras contenant 8 et/ou 10 atomes de carbone.

4. Éthers mixtes selon la revendication 1, pouvant être obtenus par une réaction de condensation de
a) 25 à 35 % en poids d'alcools gras non ramifiés ayant une longueur de chaîne de 12 à 18 atomes de carbone, et
b) 33 à 50 % en poids d'alcool isostéarylique, et 25 à 35 % en poids d'un alcool gras ayant une longueur de chaîne de 20 et/ou 22 atomes de carbone.

5. Éthers mixtes selon les revendications 1 à 4, **caractérisés en ce qu'**ils présentent une plage de fusion comprise entre -22 °C et +55 °C, la largeur de la plage de fusion comprenant au moins 50 degrés de température et le maximum de la plage de fusion se situant à 35 ± 10 °C.

6. Éthers mixtes selon les revendications 1 à 5, **caractérisés en ce que** le composant a) est constitué d'alcools gras saturés non ramifiés, ayant la distribution de chaînes suivante :
C12 de 48 à 58 % en poids
C14 de 18 à 24 % en poids
C16 de 8 à 12 % en poids
C18 de 11 à 15 % en poids.

7. Éthers mixtes selon les revendications 1 à 6, **caractérisés en ce que** le composant c) est le n-docosanol.

8. Éthers mixtes selon les revendications 1 à 5, **caractérisés en ce que** la réaction de condensation est réalisée à des températures de 200 à 250 °C.

9. Éthers mixtes selon les revendications 1 à 5, **caractérisés en ce que** de l'acide sulfonique et/ou de l'acide méthane-sulfonique sont utilisés lors de la réaction de condensation, éventuellement en combinaison avec des acides phosphoriques.

10. Préparations cosmétiques et/ou pharmaceutiques contenant des éthers mixtes selon l'une quelconque des revendications 1 à 9.

11. Utilisation d'éthers mixtes selon l'une quelconque des revendications 1 à 9 en tant que substitut de vaseline dans des préparations cosmétiques ou pharmaceutiques.
